# EUROPEAN PATENT SPECIFICATION

(11) **EP 3 260 527 B1**
(45) Date of publication and mention of the grant of the patent: **14.08.2019**
(21) Application number: 16752595.5
(22) Date of filing: 19.02.2016
(51) Int. Cl.: C12M 1/00, C12M 3/00, C12N 1/00

(54) **CELL CULTURE DEVICE AND METHOD FOR REPLACING CULTURE MEDIUM**
ZELLKULTURVORRICHTUNG UND VERFAHREN ZUM ERSETZEN EINES KULTURMEDIUMS
DISPOSITIF DE CULTURE CELLULAIRE ET PROCÉDÉ DE REMPLACEMENT DE MILIEU DE CULTURE

(30) Priority: 20.02.2015 JP 2015031956
(43) Date of publication of application: 27.12.2017
(73) Proprietor: Sinfonia Technology Co., Ltd., Tokyo 105-8564 (JP)
(72) Inventor: SHIRAIWA Hirotsugu, Tokyo 107-6325 (JP)
(74) Representative: Diehl & Partner GbR
(86) International application number: PCT/JP2016/054919
(87) International publication number: WO 2016/133209

(56) References cited:
- WO-A1-2008/133874
- JP-A- 2002 262 856
- JP-A- 2010 104 299
- JP-A- 2010 524 498
- JP-A- 2015 062 383
- US-A1- 2011 136 225
- DATABASE WPI Week 201270 Thomson Scientific, London, GB; AN 2012-N24841 XP002784784, -& WO 2012/141055 A1 (HITACHI LTD) 18 October 2012 (2012-10-18)
- DATABASE WPI Week 200547 Thomson Scientific, London, GB; AN 2005-462258 XP002784806, -& JP 2005 168384 A (OLYMPUS CORP) 30 June 2005 (2005-06-30)

## Description

### TECHNICAL FIELD

The present disclosure relates to a cell culture device for culturing cells. Moreover, the present disclosure relates to a method for replacing a culture medium using a cartridge for culture medium replacement use.

### BACKGROUND

In the related art, there is known a culture device used for culturing cells. In such a culture device, a culture medium replacement mechanism for periodically replacing a liquid culture medium inside a culture container is usually installed in order to prevent gradual deterioration of an internal culture environment of the culture container. As an example of the culture medium replacement mechanism, for example, Patent Document 1 is disclosed. In Patent Document 1, there is disclosed a mechanism in which a culture container placed on a culture medium replacement stage (container mounting stand), a culture medium storage container and a collection container are connected by different tubes, a new culture medium is supplied to the culture container from the culture medium storage container via one tube, and an old culture medium is collected from the culture container to the collection container via the other tube.

Patent Document 2 discloses a device and a method for supplying a cell fluid to a culture vessel *via* a channel, comprising introducing a cell bag and a portion of the channel arranged in a transport jig into a clean space, whereupon the cells and medium are supplied into the channel using the cell bag. A supply port is then closed.

### [Prior Art Documents]

### Patent Documents

Patent Document 1: Japanese laid-open publication No. 2008-271850
Patent Document 2: International application WO 2012/141055 A1

However, in the culture medium replacement mechanism provided in the related art, other devices such as a culture medium storage container, a collection container, a culture medium temperature regulator and a culture medium time adjustor are concentrated around the culture medium replacement stage. Therefore, due to physical limitations, a liquid flow path connecting the culture container and the culture medium storage container and the like becomes redundant. In addition, protein gradually adheres to an inner wall of the liquid flow path when in use, thereby contaminating the liquid flow path. Thus, it is necessary to periodically replace the liquid flow path. Since the liquid flow path is a tube, it is difficult to automate the replacement. It is also difficult to ensure the workability due to the concentration of devices.

Furthermore, in the culture media replacement mechanism provided in the related art, it is not possible to start a treatment on a subsequent culture container until a series of treatments such as culture medium supply, culture medium replacement, culture medium collection and flow path cleaning are completed for one culture container. Thus, the operation time in the case of treating a plurality of culture containers is prolonged.

The present description describes a cell culture device, a cartridge for culture medium replacement use and a method for replacing a culture medium, which are capable of shortening and automatically replacing a liquid flow path.

### SUMMARY

According to one embodiment of the present invention, there is provided a cell culture device, including: an incubator part configured to accommodate a closed-system culture container; a cartridge for culture medium replacement use having a liquid supply flow path and a liquid collection flow path, a culture medium supply part configured to supply a liquid culture medium to the liquid supply flow path of the cartridge for culture medium replacement use; a culture medium replacement part configured to cause the liquid culture medium existing in the liquid supply flow path to flow into the culture container and to cause the liquid culture medium existing in the culture container to flow out to the liquid collection flow path in a state in which the cartridge for culture medium replacement use is connected to the culture container; and a culture medium collection part configured to collect the liquid culture medium from the liquid collection flow path of the cartridge for culture medium replacement use, wherein the cartridge for culture medium replacement use is removably attachable to the closed-system culture container and is movable between the culture medium supply part, the culture medium replacement part and the culture medium collection part.

The device according to the present disclosure may further include: a cartridge conveying part configured to move the cartridge for culture medium replacement use between the culture medium supply part, the culture medium replacement part and the culture medium collection part.

The device according to the present disclosure may further include: a flow path cleaning part configured to clean the liquid supply flow path and the liquid collection flow path of the cartridge for culture medium replacement use, wherein the cartridge for culture medium replacement use may move between the culture medium supply part, the culture medium replacement part, the culture medium collection part and the flow path cleaning part.

In the device according to the present disclosure, the culture medium replacement part may be installed adjacent to the incubator part, and a culture medium replacement in the culture container may be performed via the cartridge for culture medium replacement use while keeping the culture container accommodated in the incubator part.

In the device according to the present disclosure, a culture medium analysis part configured to analyze a collected liquid culture medium may be installed in the culture medium collection part.

The device according to the present disclosure may further include: a cartridge storage part configured to store a plurality of unused cartridges for culture medium replacement use; and a cartridge collection part configured to collect a used cartridge for culture medium replacement use.

In the device according to the present disclosure, the liquid supply flow path may include a first inflow port connectable to the culture medium supply part, a first outflow port connectable to an inflow port of the culture container, a liquid storage chamber configured to bring the first inflow port and the first outflow port into communication with each other and a ventilation port communicating with the liquid storage chamber, and the liquid collection flow path may include a second inflow port connectable to an outflow port of the culture container, a second outflow port connectable to the culture medium collection part and a curved flow path configured to bring the second inflow port and the second outflow port into communication with each other, the curved flow path including a plurality of bent portions or curved portions.

In the device according to the present disclosure, the curved flow path may have a serpentine shape or a spiral shape.

In the device according to the present disclosure, the first outflow port and the second inflow port may be formed adjacent to each other.

A cartridge for culture medium replacement use may include a liquid supply flow path; and a liquid collection flow path, wherein the cartridge is removably attachable to a closed-system culture container and is movable between a culture medium supply part, a culture medium replacement part and a culture medium collection part of a cell culture device.

In the cartridge the liquid supply flow path may include a first inflow port connectable to the culture medium supply part, a first outflow port connectable to an inflow port of the culture container, a liquid storage chamber configured to bring the first inflow port and the first outflow port into communication with each other and a ventilation port communicating with the liquid storage chamber, and the liquid collection flow path may include a second inflow port connectable to an outflow port of the culture container, a second outflow port connectable to the culture medium collection part and a curved flow path configured to bring the second inflow port and the second outflow port into communication with each other, the curved flow path including a plurality of bent portions or curved portions.

In the cartridge the curved flow path may have a serpentine shape or a spiral shape.

In the cartridge the first outflow port and the second inflow port may be formed adjacent to each other.

A method for replacing a culture medium according to the present disclosure, including: a culture medium supply step of connecting a cartridge for culture medium replacement use having a liquid supply flow path and a liquid collection flow path to a culture medium supply part, supplying a liquid culture medium from the culture medium supply part to the liquid supply flow path, and separating the cartridge for culture medium replacement use from the culture medium supply part; a culture medium replacement step of connecting the cartridge for culture medium replacement use to a closed-system culture container, causing the liquid culture medium existing in the liquid supply flow path to flow into the culture container, causing the liquid culture medium existing in the culture container to flow out to the liquid collection flow path, and separating the cartridge for culture medium replacement use from the culture container; and a culture medium collection step of connecting the cartridge for culture medium replacement use to a culture medium collection part, collecting the liquid culture medium from the liquid collection flow path to the culture medium collection part, and separating the cartridge for culture medium replacement use from the culture medium collection part.

The method according to the present disclosure may further include a cleaning step of cleaning the liquid supply flow path and the liquid collection flow path of the cartridge for culture medium replacement use after the culture medium collection step.

In the method according to the present disclosure, the culture medium replacement step may be performed in a state in which the culture container is accommodated in an incubator part.

The method according to the present disclosure may further include a culture medium analysis step of analyzing a collected liquid culture medium after the culture medium collection step.

A cartridge for culture medium replacement use capable of accommodating a predetermined amount of liquid culture medium moves between a culture medium supply part, a culture medium replacement part and a culture medium collection part. This makes it possible to perform a culture medium replacement in a state in which a culture container is separated from the culture medium supply part and the culture medium collection part. Therefore, it is possible to shorten a liquid flow path. In addition, the liquid flow path is formed in the cartridge for culture medium replacement use, namely a cartridge-type part. This makes it easy to automate the replacement of the liquid flow path.

### BRIEF DESCRIPTION OF THE DRAWINGS

FIG. 1 is a schematic top plan view showing a configuration of an automatic culture system according to an embodiment of the present disclosure.
FIG. 2 is a control block diagram of an automatic culture system according to an embodiment of the present disclosure.
FIG. 3 is a front view showing a container conveying part used in an embodiment of the present disclosure.
FIG. 4 is a schematic plan view showing a configuration in the vicinity of a liquid storage supply part and an incubator part used in an embodiment of the present disclosure.
FIG. 5A is a schematic view showing a configuration of a cartridge for culture medium replacement use utilized in an embodiment of the present disclosure.
FIG. 5B is a schematic view showing a configuration of a first modification of the cartridge for culture medium replacement use.
FIG. 5C is a schematic view showing a configuration of a second modification of the cartridge for culture medium replacement use.
FIG. 6A is a schematic plan view showing a structure of a valve of the cartridge for culture medium replacement use shown in FIG. 5A.
FIG. 6B is a sectional view taken along line A-A of the valve shown in FIG. 6A.
FIG. 7A is a view corresponding to FIG. 6B and explaining an operation of the valve.
FIG. 7B is a view corresponding to FIG. 6B and explaining the operation of the valve.
FIG. 7C is a view corresponding to FIG. 6B and explaining the operation of the valve.
FIG. 8A is a view corresponding to FIG. 5A and explaining a culture medium supply step.
FIG. 8B is a view corresponding to FIG. 5A and explaining the culture medium supply step.
FIG. 9A is a view corresponding to FIG. 5A and explaining a culture medium replacement step.
FIG. 9B is a view corresponding to FIG. 5A and explaining the culture medium replacement step.
FIG. 10A is a view corresponding to FIG. 5A and explaining a culture medium collection step.
FIG. 10B is a view corresponding to FIG. 5A and explaining the culture medium collection step.
FIG. 11A is a view corresponding to FIG. 5A and explaining a flow path cleaning step.
FIG. 11B is a view corresponding to FIG. 5A and explaining the flow path cleaning step.
FIG. 11C is a view corresponding to FIG. 5A and explaining the flow path cleaning step.
FIG. 11D is a view corresponding to FIG. 5A and explaining the flow path cleaning step.
FIG. 12A is an internal top view showing a configuration of a first modification of a transport container.
FIG. 12B is an internal side view showing the configuration of the first modification of the transport container.
FIG. 13 is a view corresponding to FIG. 12B and explaining an operation of the transport container.
FIG. 14 is an internal top view corresponding to FIG. 12A and showing a configuration of a second modification of the transport container.
FIG. 15A is a schematic plan view showing a configuration of a modification example of the cell culture device.
FIG. 15B is a sectional view taken along line B-B of the cell culture device in FIG. 15A.
FIG. 15C is a sectional view taken along line C-C of the cell culture device in FIG. 15A.
FIG. 15D is a left side view of the cell culture device shown in FIG. 15A.

### DETAILED DESCRIPTION

Embodiments of the present disclosure will now be described in detail with reference to the accompanying drawings. Throughout the drawings attached hereto, for the convenience of illustration and ease of understanding, the scales, the aspect ratios and the like are changed and exaggerated from actual embodiments.

The culture device according to the present embodiment may be used for culturing all kinds of cells and may be used when culturing various cells including pluripotent stem cells such as (human) iPS cells, (human) ES cells or the like, chondrocytes such as bone marrow stromal cells (MSCs) or the like, dendritic cells, and so forth. In the present description, hereinafter, an automatic culture system for automatically culturing iPS cells will be described. However, it should be noted that this is nothing more than an example. In other words, it should be noted that the culture device according to the present embodiment may be used even if cells are not automatically cultured as in the present embodiment.

### <Overall Configuration>

First, the configuration of the automatic culture system will be described.

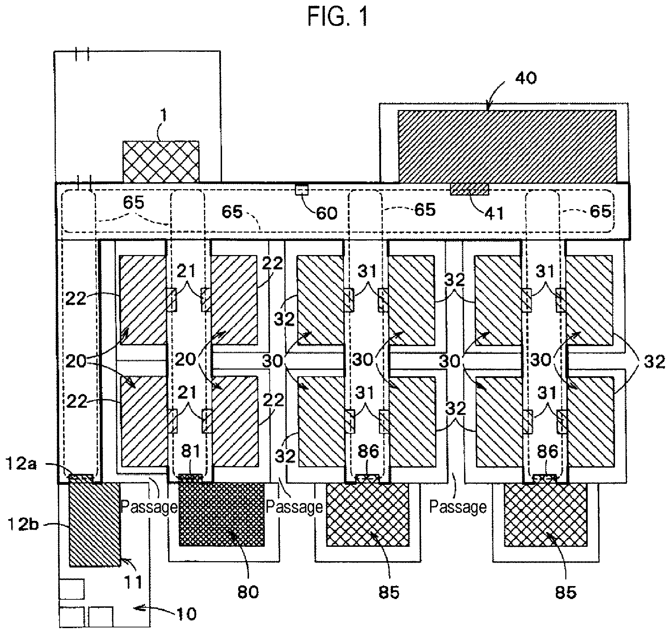

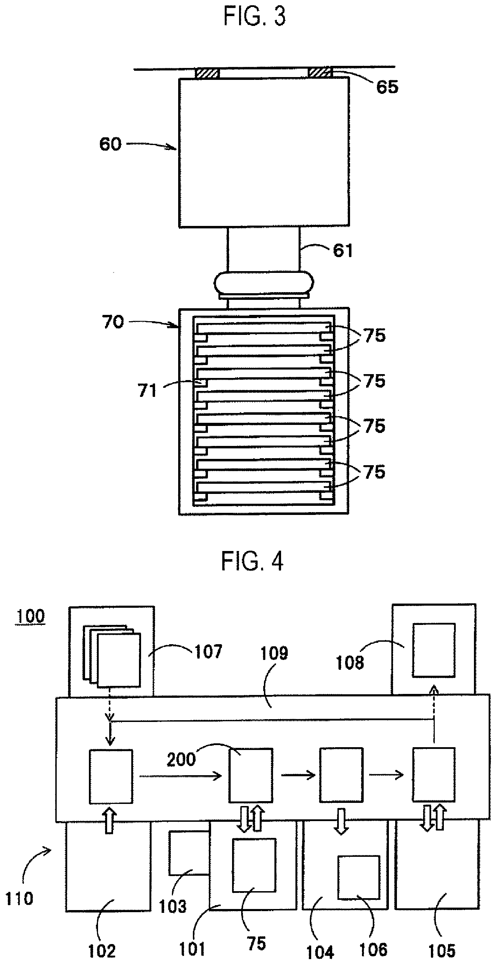

As shown in FIG. 1, the automatic culture system includes a raw material storage device 10 configured to store raw material cells, a container transfer part 60 configured to transfer a transport container 70 (*see* FIG. 3) in which cells or the like are accommodated in a sealed state, and cell culture devices 20 and 30 configured to receive the transport container 70 transferred by the container transfer part 60, takes out a closed-system culture container 75 in which cells are accommodated from the transport container 70, and cultures the cells contained in the taken-out culture container 75.

The mode using iPS cells as mentioned above will be described. Thus, the raw material storage device 10 includes an iPS cell establishing device 11 configured to establish iPS cells. In addition, the raw material storage device 10 includes a unit thermostat bath, a centrifugal separator, an automatic blood cell counter, an automatic magnetic cell separator, a flow cytometer, a gene introduction device, and the like.

The cell culture devices 20, 30 include a plurality of (four shown in FIG. 1) iPS cell automatic culture devices 20 configured to automatically culture iPS cells, and a plurality of (eight shown in FIG. 1) differentiated cell automatic culture devices 30 configured to automatically culture differentiated cells differentiated from the iPS cells. When merely referred to as "cell culture device", it means the iPS cell automatic culture device 20, the differentiated cell automatic culture device 30, or both the iPS cell automatic culture device 20 and the differentiated cell automatic culture device 30. Unless specifically mentioned otherwise, when merely referred to as "cells", it means raw material cells such as somatic cells forming the basis of iPS cells or the like, iPS cells, differentiated cells, or two or all of the raw material cells, the iPS cells and the differentiated cells.

As shown in FIG. 3, the transport container 70 includes a plurality of (eight, in FIG. 3) shelves 71 configured to support the plurality of culture containers 75. Each of the culture containers 75 is placed on each of the shelves 71. The transport container 70 is transferred by the container transfer part 60 in a state in which the plurality of culture containers 75 is accommodated in the transport container 70.

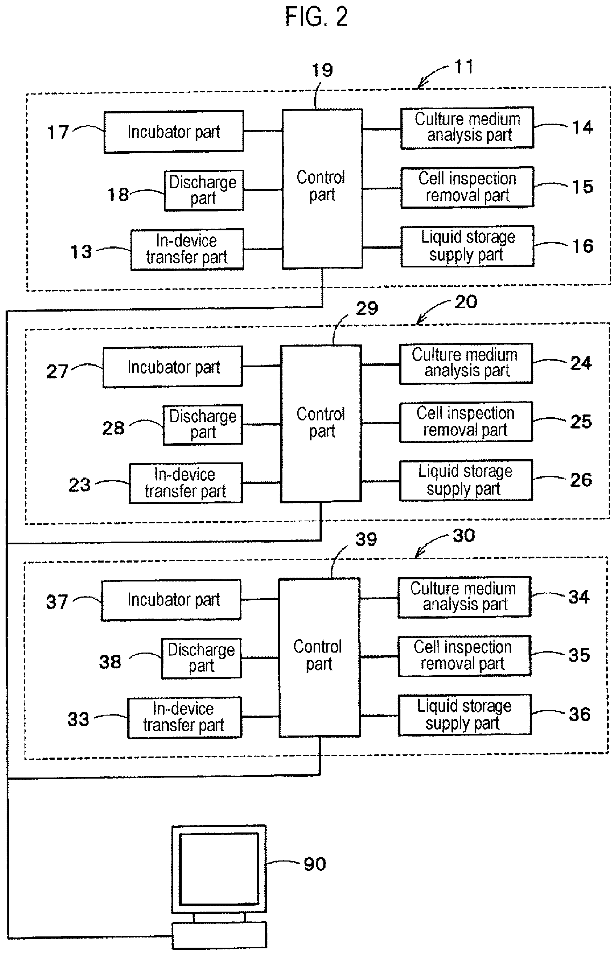

As illustrated in FIG. 2, the iPS cell automatic culture device 20 includes a housing 22 illustrated in FIG. 1, a culture medium analysis part 24 configured to analyze liquid culture medium components that vary with the culture of iPS cells, a cell inspection removal part 25 configured to inspect the iPS cells and performs removal of the iPS cells having a bad state, a liquid storage supply part 26 configured to store and supply a liquid containing a liquid culture medium or a proteolytic enzyme and to perform a pre-treatment before iPS cells are seeded, to seed iPS cells or to collect iPS cells, an incubator part 27 configured to hold the culture container 75 and automatically adjust one or all of a temperature, a humidity and a gas concentration, and a discharge part 28 configured to discharge downward from the housing 22 a waste liquid containing a used liquid culture medium, a used cleaning liquid, a used reagent or the like, which is used in the iPS cell automatic culture device 20. Furthermore, the iPS cell automatic culture device 20 includes an in-device transfer part 23 configured to transfer the culture container 75 or the like inside the iPS cell automatic culture device 20. The liquid storage supply part 26 described above also has a function of inverting the culture container 75 to be upside down. Incidentally, in the case where the closed-system culture container 75 is used as in the present embodiment, it is not necessarily required to manage the internal humidity of the incubator part 27 at a strict level as compared with the case of using an open-system culture container. This makes it possible to simplify the management of a cell culture environment. By employing the closed-system culture container 75 of this type, there is no fear that contamination from the air occurs. Furthermore, the transfer becomes easy.

The liquid storage supply part 26 described above appropriately supplies a liquid culture medium from an inflow port (not shown) into the culture container 75, thereby automatically replacing an old liquid culture medium existing in the culture container 75 with a new liquid culture medium. Based on the information of the iPS cells acquired, the cell inspection removal part 25 selectively peels off defective iPS cells from an ECM (Extracellular Matrix) coated on the surface of a film (not shown) of the culture container 75. Thereafter, the liquid storage supply part 26 supplies a liquid culture medium from the inflow port into the culture container 75, thereby pushing out floating defective iPS cells from the culture container 75 through an outflow port (not shown). As the method of selectively peeling off the iPS cells existing in the culture container 75, it may be possible to use a method of irradiating ultrasonic waves or light onto the iPS cells or a method of applying a physical force from outside the culture container 75. When using this method, it may be possible to use a proteolytic enzyme in combination.

Furthermore, the liquid storage supply part 26 appropriately supplies a proteolytic enzyme from the inflow port into the culture container 75, thereby peeling off the iPS cells from the ECM coated on the surface of a film of the culture container 75. Thereafter, the liquid storage supply part 26 supplies a liquid culture medium from the inflow port into the culture container 75, whereby floating iPS cells are pushed out from the culture container 75 through the outflow port. The iPS cells thus pushed out are diluted into a suspension and are then accommodated (seeded) in a plurality of other culture containers 75. In this way, the iPS cell automatic culture device 20 automatically performs the subculture of the iPS cells.

The internal temperature of the iPS cell automatic culture device 20 is adjusted by the incubator part 27 so that the internal temperature becomes, for example, about 37 degrees C. Furthermore, the gas concentration in the iPS cell automatic culture device 20 is adjusted by the incubator part 27 by appropriately adding carbon dioxide or nitrogen. If necessary, the humidity may be adjusted by the incubator part 37 so as to become about 100%.

As illustrated in FIG. 2, the differentiated cell automatic culture device 30 includes a housing 32 illustrated in FIG. 1, a culture medium analysis part 34 configured to analyze liquid culture medium components that vary with the culturing of differentiated cells, a cell inspection removal part 35 configured to inspect the differentiated cells and perform removal of the differentiated cells having a bad state, a liquid storage supply part 36 configured to store and supply a liquid containing a liquid culture medium or a proteolytic enzyme and to perform a pre-treatment before differentiated cells are seeded, seed differentiated cells or collect differentiated cells, an incubator part 37 configured to hold the culture container 75 and automatically adjust one or all of a temperature, a humidity and a gas concentration, and a discharge part 38 configured to discharge downward from the housing 32 a waste liquid containing a used liquid culture medium, a used cleaning liquid, a used reagent or the like, which is used in the differentiated cell automatic culture device 30. Furthermore, the differentiated cell automatic culture device 30 includes an in-device transfer part 33 configured to transfer the culture container 75 or the like within the differentiated cell automatic culture device 30.

The liquid storage supply part 36 described above appropriately supplies a liquid culture medium from the inflow port into the culture container 75, thereby automatically replacing an old liquid culture medium existing in the culture container 75 with a new liquid culture medium. Based on the information of the differentiated cells acquired, the cell inspection removal part 35 selectively peels off defective differentiated cells from the ECM coated on the surface of a film 77 of the culture container 75. Thereafter, the liquid storage supply part 36 supplies a liquid culture medium from the inflow port into the culture container 75, thereby pushing out floating defective differentiated cells from the culture container 75 through the outflow port. As the method of selectively peeling off the differentiated cells existing in the culture container 75, it may be possible to use a method of irradiating ultrasonic waves or light onto the differentiated cells or a method of applying a physical force from outside the culture container 75. When using this method, it may be possible to use a proteolytic enzyme in combination.

Furthermore, the liquid storage supply part 36 appropriately supplies a proteolytic enzyme from the inflow port into the culture container 75, thereby peeling off the differentiated cells from the ECM coated on the surface of the film of the culture container 75. Thereafter, the liquid storage supply part 36 supplies a liquid culture medium from the inflow port into the culture container 75, whereby floating differentiated cells are pushed out from the culture container 75 through the outflow port. The differentiated cells thus pushed out are diluted into a suspension and are then accommodated (seeded) within a plurality of other culture containers 75. In this way, the differentiated cell automatic culture device 30 automatically performs the subculture of the differentiated cells.

The internal temperature of the differentiated cell automatic culture device 30 is adjusted by the incubator part 37 so that the internal temperature becomes, for example, about 37 degrees C. Furthermore, the gas concentration within the differentiated cell automatic culture device 30 is adjusted by the incubator part 37 by appropriately adding carbon dioxide or nitrogen. When inducing differentiation, the liquid storage supply part 36 of the differentiated cell automatic culture device 30 may supply a liquid culture medium including a differentiation-inducing factor. If necessary, the humidity may be adjusted by the incubator part 37 so as to become about 100%.

As illustrated in FIG. 2, the iPS cell automatic culture device 20 includes a control part 29 which is connected to each of the culture medium analysis part 24, the cell inspection removal part 25, the liquid storage supply part 26, the incubator part 27, the discharge part 28 and the in-device transfer part 23 so as to make communication therewith and which is configured to control them. The control part 29 has a function of, with respect to the iPS cell automatic culture device 20, managing the status, managing the log, managing the culture schedule, or serving as a user interface. Furthermore, the differentiated cell automatic culture device 30 includes a control part 39 which is connected to each of the culture medium analysis part 34, the cell inspection removal part 35, the liquid storage supply part 36, the incubator part 37, the discharge part 38 and the in-device transfer part 33 so as to make communication therewith and which is configured to control them. The control part 29 has a function of, with respect to the differentiated cell automatic culture devices 30, managing the status, managing the log, managing the culture schedule, or serving as a user interface.

The iPS cell establishing device 11 is similar in configuration to the iPS cell automatic culture device 20 and the differentiated cell automatic culture device 30. That is to say, as illustrated in FIG. 2, the iPS cell establishing device 11 includes a housing 12b illustrated in FIG. 1, a culture medium analysis part 14 configured to analyze a liquid culture medium, a cell inspection removal part 15 configured to inspect the raw material cells and perform removal of the raw material cells having a bad state, a liquid storage supply part 16 configured to store and supply a liquid containing a liquid culture medium or a proteolytic enzyme, an incubator part 17 configured to automatically adjust one or all of a temperature, a humidity and a gas concentration in the housing 12b, and a discharge part 18 configured to discharge downward from the housing 12b a waste liquid containing a used liquid culture medium, a used cleaning liquid, a used reagent or the like, which is used in the iPS cell establishing device 11. Furthermore, the iPS cell establishing device 11 further includes an in-device transfer part 13 configured to transfer the culture container 75 within the iPS cell establishing device 11. Moreover, the iPS cell establishing device 11 includes a control part 19 which is connected to each of the culture medium analysis part 14, the cell inspection removal part 15, the liquid storage supply part 16, the incubator part 17, the discharge part 18 and the in-device transfer part 13 so as to make communication therewith and which is configured to control them. Each of the control parts 19, 29 and 39 is connected to an external device 90 such as, e.g., a personal computer or the like.

As illustrated in FIG. 3, the container transfer part 60 includes a holding part 61 which holds the transport container 70 while suspending downward the transport container 70. The container transfer part 60 is configured to move along a rail 65 installed in a ceiling.

As illustrated in FIG. 1, the iPS cell automatic culture device 20 includes a loading part 21 configured to load the culture container 75 from the transport container 70 therethrough. The loading part 21 may include a cell loading/unloading part (not shown) for loading the iPS cells accommodated in the culture container 75 therethrough and for unloading the cultured iPS cells therefrom, and a material loading part (not shown) for loading materials accommodated in other airtight containers therethrough. Similarly, as illustrated in FIG. 1, the differentiated cell automatic culture device 30 includes a loading part 31 configured to load the culture container 75 from the transport container 70 therethrough. The loading part 31 may include a cell loading/unloading part (not shown) for loading the iPS cells accommodated in the culture container 75 therethrough and for unloading the cultured differentiated cells therefrom, and a material loading part (not shown) for loading materials accommodated in other airtight containers therethrough. The materials may include a liquid culture medium, a reagent, a cleaning liquid, a culture plate, a vial, a filter, a needle, and so forth. Furthermore, as illustrated in FIG. 1, the iPS cell establishing device 11 includes a loading part 12a configured to load the transport container 70 therethrough.

As illustrated in FIG. 1, the automatic culture system includes a sterilizing device 1 for sterilizing the interior of the transport container 70, an iPS cell analysis device 80 which receives the iPS cells cultured in the iPS cell automatic culture device 20 from the loading part 81 at a predetermined timing and which inspects the iPS cells thus received, a differentiated cell analysis device 85 which receives the differentiated cells cultured in the differentiated cell automatic culture devices 30 from the loading part 86 at a predetermined timing and which inspects the differentiated cells thus received, and a freezing storage device 40 which receives the iPS cells, the differentiated cells or both cultured in the automatic culture devices 20 and 30 from the loading part 41 and which freezes and stores the iPS cells, the differentiated cells or both thus received. A plurality of freezing storage devices 40 may be provided. The entire room may be cooled and may serve as a freezer. In the case where the plurality of freezing storage devices 40 are installed in the room or in the case where the room itself serves as a freezer, the rail 65 may be installed in the ceiling of the room so that the container transfer part 60 can move along the rail 65.

One example of the sterilizing device 1 described above may include a sterilizing device which sterilizes the interior of the transport container 70 by supplying a sterilizing gas such as a hydrogen peroxide gas or a high-temperature gas into the transport container 70. Another example of the sterilizing device 1 may include a sterilizing device which sterilizes the interior of the transport container 70 by irradiating, for example, γ rays or ultraviolet rays from the outside while keeping the transport container 70 in a sealed state. In addition, before the transport container 70 is loaded from the outside, the interior of the transport container 70 may be sterilized using, for example, γ rays or ultraviolet rays. There may be a case where the liquid culture medium or the like contains protein or the like which is broken by γ rays or ultraviolet rays. In this case, it is desirable that sterilization is performed by a sterilizing gas such as a hydrogen peroxide gas, a high-temperature gas or the like.

### <Configuration around Liquid Storage Supply Part and Incubator Part>

Next, the configuration around the liquid storage supply part and the incubator part will be described.

In the following descriptions, one, two or all of the liquid storage supply part 16 of the iPS cell establishing device 11, the liquid storage supply part 26 of the iPS cell automatic culture devices 20 and the liquid storage supply part 36 of the differentiated cell automatic culture devices 30 will be referred to as a "liquid storage supply part 110." The liquid storage supply part 16 of the iPS cell establishing device 11, the liquid storage supply part 26 of the iPS cell automatic culture devices 20 and the liquid storage supply part 36 of the differentiated cell automatic culture devices 30 have the same configuration.

In the following descriptions, one, two or all of the incubator part 17 of the iPS cell establishing device 11, the incubator part 27 of the iPS cell automatic culture device 20 and the incubator part 37 of the differentiated cell automatic culture device 30 will be referred to as an "incubator part 101." The incubator part 17 of the iPS cell establishing device 11, the incubator part 27 of the iPS cell automatic culture device 20 and the incubator part 37 of the differentiated cell automatic culture device 30 have the same configuration.

In the following descriptions, one, two or all of the culture medium analysis part 14 of the iPS cell establishing device 11, the culture medium analysis part 24 of the iPS cell automatic culture devices 20 and the culture medium analysis part 34 of the differentiated cell automatic culture devices 30 will be referred to as a "culture medium analysis part 106." The culture medium analysis part 14 of the iPS cell establishing device 11, the culture medium analysis part 24 of the iPS cell automatic culture devices 20 and the culture medium analysis part 34 of the differentiated cell automatic culture devices 30 have the same configuration.

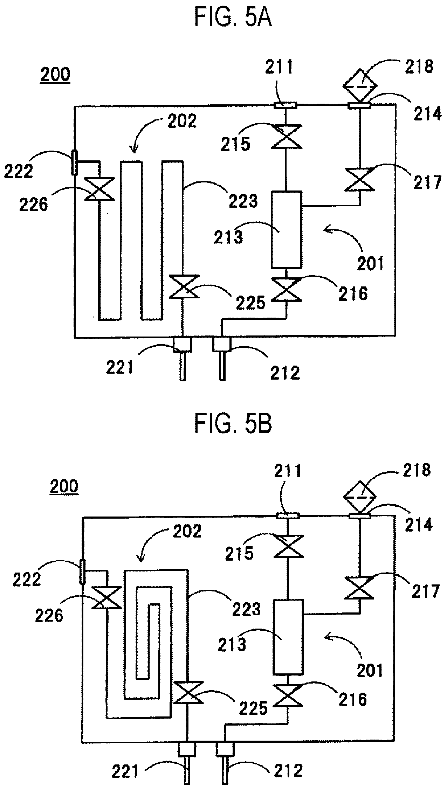

FIG. 4 is a schematic plan view showing a configuration around the liquid storage supply part 110 and the incubator part 101. As shown in FIG. 4, the liquid storage supply part 110 includes a cartridge for culture medium replacement use 200 having a liquid supply flow path 201 and a liquid collection flow path 202 (*see* FIG. 5A) and detachably installed in the culture container 75, a culture medium supply part 102 configured to supply a liquid culture medium to the liquid supply flow path 201 of the cartridge for culture medium replacement use 200, a culture medium replacement part 103 configured to cause the liquid culture medium inside the liquid supply flow path 201 to flow into the culture container 75 and cause the culture medium inside the culture container 75 to flow out toward the liquid collection flow path 202 in a state in which the cartridge for culture medium replacement use 200 is connected to the culture container 75, a culture medium collection part 104 configured to collect the liquid culture medium from the liquid collection flow path 202 of the cartridge for culture medium replacement use 200, and a cartridge conveying part 109 configured to move the cartridge for culture medium replacement use 200 between the culture medium supply part 102 and the culture medium replacement part 103 and the culture medium collection part 104. In FIG. 4, thin arrows indicate the direction of movement of the cartridge for culture medium replacement use 200, and thick arrows indicate the direction of movement of the liquid culture medium.

Furthermore, a culture medium analysis part 106 configured to analyze the components of the collected liquid culture medium is installed in the culture medium collection part 104.

Moreover, in the present embodiment, as shown in FIG. 4, there is installed a flow path cleaning part 105 configured to clean the liquid supply flow path 201 and the liquid collection flow path 202 of the cartridge for culture medium replacement use 200. The cartridge conveying part 109 is adapted to move the cartridge for culture medium replacement use 200 between the culture medium supply part 102, the culture medium replacement part 103, the culture medium collection part 104 and the flow path cleaning part 105.

Furthermore, in the present embodiment, as shown in FIG. 4, there are further installed a cartridge storage part 107 configured to store a plurality of unused cartridges for culture medium replacement use 200 and a cartridge collection part 108 configured to collect the used cartridges for culture medium replacement use 200. The cartridge conveying part 109 is configured to periodically discharge the used cartridge for culture medium replacement use 200 to the cartridge collection part 108 and periodically take out the unused cartridge for culture medium replacement use 200 from the cartridge storage part 107

As the cartridge conveying part 109, it may be possible to use, for example, a conveying robot which has a hand capable of gripping a cartridge-type component and which is well-known in the related art.

### <Configuration of Cartridge for Culture Medium Replacement Use>

Next, a configuration of the cartridge for culture medium replacement use 200 will be described in detail with reference to FIG. 5A.

In the present embodiment, as shown in FIG. 5A, the liquid supply flow path 201 includes a first inflow port 211 connectable to the culture medium supply part 102, a first outflow port 212 connectable to the inflow port of the culture container 75, a liquid storage chamber 213 configured to bring the first inflow port 211 and the first outflow port 212 into communication with each other, and a ventilation port 214 communicating with the liquid storage chamber 213. A filter 218 for preventing intrusion of extraneous matter from ambient air is attached to the ventilation port 214.

The capacity of the liquid storage chamber 213 is about 1 to 1.5 times as large as the capacity of the culture container 75 and is preferably larger than the capacity of the culture container 75. Specifically, the capacity of the liquid storage chamber 213 is, for example, 30 ml. As shown in FIG. 5A, an openable/closable valve 215 is installed in a flow path formed between the first inflow port 211 and the liquid storage chamber 213. An openable/closable valve 216 is installed in a flow path formed between the liquid storage chamber 213 and the first outflow port 212. Further, an openable/closable valve 217 is installed in a flow path formed between the liquid storage chamber 213 and the ventilation port 214. By closing the respective valves 215, 216 and 217, the liquid storage chamber 213 is hermetically sealed.

On the other hand, the liquid collection flow path 202 includes a second inflow port 221 connectable to the outflow port of the culture container 75, a second outflow port 222 connectable to the culture medium collection part 104, and a curved flow path 223 configured to bring the second inflow port 221 and the second outflow port 222 into communication with each other. The curved flow path 223 includes a plurality of bent portions or curved portions. By including the bent portions or curved portions, the curved flow path 223 can be formed to have a small flow path diameter and a large flow path length. Thus, the old culture medium flowing out from the culture container 75 can be collected so as not to be mixed with the new culture medium in the flow path.

In the example shown in FIG. 5A, the curved flow path 223 has a serpentine shape. However, as long as the curved flow path 223 can collect the old medium so as not to be mixed with the new culture medium in the flow path, it is not necessarily required for the curved flow path 223 to have a serpentine shape. For example, as shown in FIG. 5B, the curved flow path 223 may have a spiral shape.

The capacity of the curved flow path 223 is about 1 to 1.5 times as large as the capacity of the culture container 75 and is preferably larger than the capacity of the culture container 75. Specifically, the capacity of the curved flow path 223 is, for example, 30 ml. In order to suppress the generation of a turbulent flow in the curved flow path 223, the diameter of the curved flow path 223 may be 4 mm or less. As shown in FIG. 5A, an openable/closable valve 225 is installed in a flow path formed between the second inflow port 221 and the curved flow path 223. An openable/closable valve 226 is installed in a flow path formed between the curved flow path 223 and the second outflow port 222. By closing the respective valves 225 and 226, the curved flow path 223 is hermetically sealed.

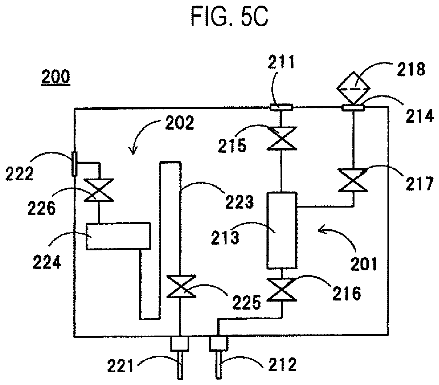

In the example shown in FIGS. 5A and 5B, the entire curved flow path extending from the second inflow port 221 to the second outflow port 222 is formed as a narrow curved flow path 223. However, as shown in FIG. 5C, within a range where no mixing of liquids occurs between the old culture medium and the new culture medium (for example, within 1/2 of the collection capacity), a portion of the flow path extending from the second inflow port 221 to the second outflow port 222 may be formed as a tank-shaped flow path 224 and the remaining portion of the flow path may be formed as a narrow curved flow path 223. Considering the conductance of the flow path from the second inflow port 221 to the second outflow port 222, it is preferable that a portion of the flow path is formed as the tank-shaped flow path 224.

The first outflow port 212 and the second inflow port 221 are formed adjacent to each other. Thus, the first outflow port 212 and the second inflow port 221 can be easily connected to the inflow port and the outflow port of the culture container 75, respectively.

Hereinafter, one, two or more or all of the valve 215 between the first inflow port 211 and the liquid storage chamber 213, the valve 216 between the liquid storage chamber 213 and the first outflow port 212, the valve 217 between the liquid storage chamber 213 and the ventilation port 214, the valve 225 between the second inflow port 221 and the curved flow path 223, and the valve 226 between the curved flow path 223 and the second outflow port 222 will be referred to as a "valve 240." In the present embodiment, the valves 215, 216, 217, 225 and 226 have the same configuration.

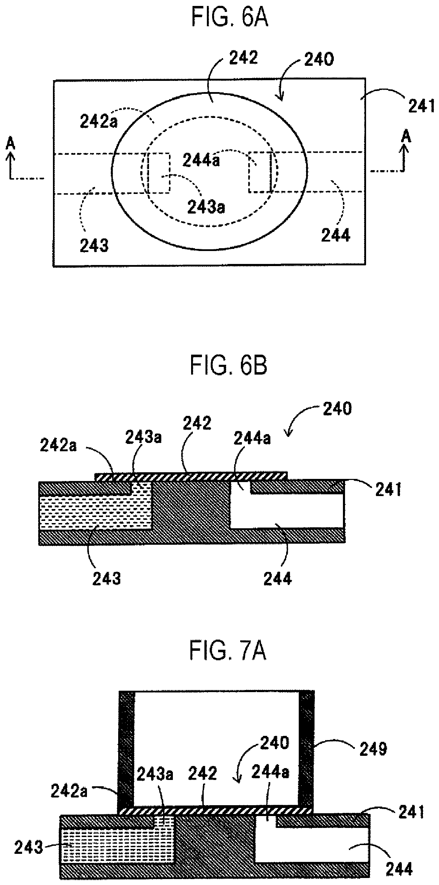

FIG. 6A is a schematic plan view showing a structure of the valve 240, and FIG. 6B is a sectional view of the valve 240 taken along line A-A in FIG. 6A.

As shown in FIGS. 6A and 6B, the valve 240 includes a main body 241 having a pair of flow paths 243 and 244 formed therein, and a sheet-like valve body 242 fixed to an upper surface of the main body 241. The valve body 242 is made of an elastomer and is bonded to the upper surface of the main body 241 at the peripheral portion 242a thereof. One end portion 243a of one flow path 243 and one end portion 244a of the other flow path 244 are opened toward the upper surface of the main body 241 at a region inward of the peripheral portion 242a of the valve body 242. Normally, a lower surface of the valve body 242 is brought into close contact with the upper surface of the main body 241 by virtue of the restoration force (elastic force) of the sheet-like valve body 242. Therefore, one end portion 243a of one flow path 243 and one end portion 244a of the other flow path 244 are closed by the valve body 242. As a result, one flow path 243 is blocked from the other flow path 244.

The operation of the valve 240 will be described with reference to FIGS. 7A to 7C.

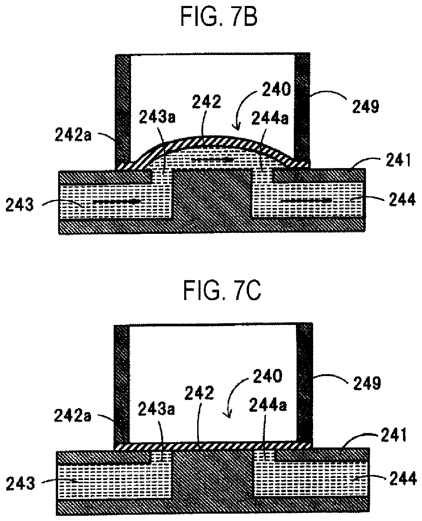

When the valve 240 is opened, first, as shown in FIG. 7A, a tubular valve driving mechanism 249 is pressed against the upper surface of the peripheral portion 242a of the valve body 242. Subsequently, as shown in FIG. 7B, the inside of the valve driving mechanism 249 is evacuated and the region inward of the peripheral portion 242a of the valve body 242 is deformed so as to bulge upward. Thus, the valve body 242 is moved away from the one end portion 243a of one flow path 243 and one end portion 244a of the other flow path 244. In the region inward of the peripheral portion 242a of the valve body 242, one flow path 243 communicates with the other flow path 244.

Subsequently, when the valve 240 is closed, as shown in FIG. 7C, the inside of the valve driving mechanism 249 returns to atmospheric pressure, and the lower surface of the valve body 242 is brought into close contact with the upper surface of the main body 241 by the restoring force of the valve body 242. Thus, one end portion 243a of one flow path 243 and one end portion 244a of the other flow path 244 are respectively closed by the valve body 242. As a result, one flow path 243 is blocked from the other flow path 244.

The cartridge for culture medium replacement use 200 configured as above can be manufactured by injection-molding a hard resin such as, for example, polystyrene or the like. In this case, the portion of the valve 240 can be formed of a hard resin and an elastomer by a two-color molding method. The method of manufacturing the cartridge for culture medium replacement use 200 is not limited to injection molding and may be manufactured by, for example, a layered modeling method using a 3D printer.

### <Culture Medium Replacement Method>

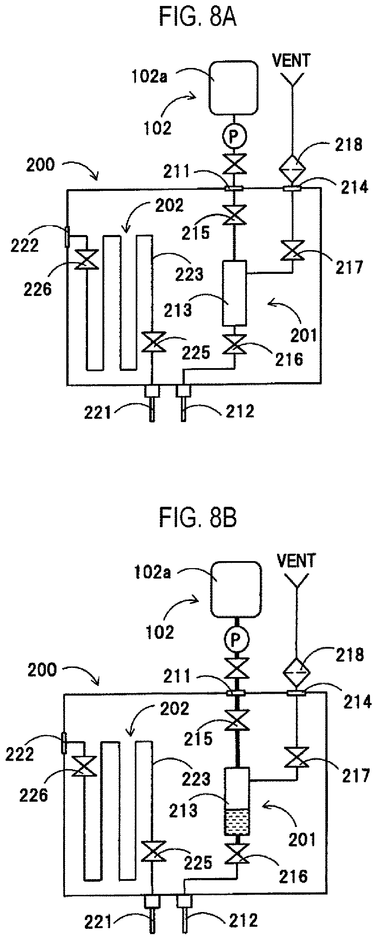

Next, a culture medium replacement method using the cartridge for culture medium replacement use 200 will be described with reference to FIG. 4 and FIGS. 8A to 11D. Thick lines in FIGS. 8A to 11D indicate flow paths through which a liquid is passing. First, as shown in FIG. 4, the cartridge conveying part 109 takes out the cartridge for culture medium replacement use 200 from the cartridge storage part 107 and moves the same to the culture medium supply part 102.

In the culture medium supply part 102, as shown in FIG. 8A, a culture medium storage container 102a is connected to the first inflow port 211 of the cartridge for culture medium replacement use 200. Then, as shown in FIG. 8B, the valve 215 between the first inflow port 211 and the liquid storage chamber 213 and the valve 217 between the liquid storage chamber 213 and the ventilation port 214 are opened. On the other hand, the valve 216 between the liquid storage chamber 213 and the first outflow port 212 is closed. In this state, a liquid culture medium is supplied from the culture medium storage container 102a via the first inflow port 211. Since the valve 216 between the liquid storage chamber 213 and the first outflow port 212 is closed, the supplied liquid culture medium is stored in the liquid storage chamber 213. Thereafter, the valve 215 between the first inflow port 211 and the liquid storage chamber 213 and the valve 217 between the liquid storage chamber 213 and the ventilation port 214 are also closed and the liquid storage chamber 213 is sealed. The valve driving mechanism 249 is installed in the culture medium supply part 102. The valves 215 and 217 operated here are opened and closed by the valve driving mechanism 249.

Subsequently, as shown in FIG. 4, by the cartridge conveying part 109, the cartridge for culture medium replacement use 200 is separated from the culture medium supply part 102 in a state in which the culture medium is stored in the liquid storage chamber 213, and is moved to the culture medium replacement part 103. The culture container 75 is accommodated in advance in the incubator part 101 adjacent to the culture medium replacement part 103.

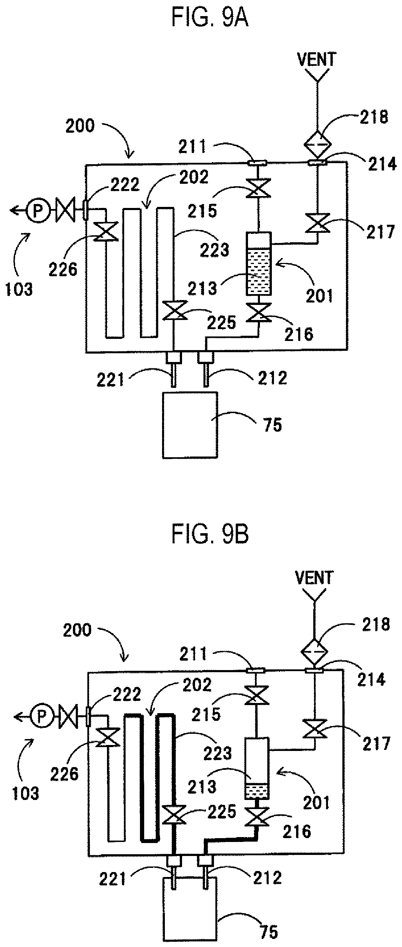

In the culture medium replacement part 103, as shown in FIG. 9A, a depressurization pump is connected to the second outflow port 222 of the cartridge for culture medium replacement use 200. In a state in which the culture container 75 is accommodated in the incubator part 101, as shown in FIG. 9B, the first outflow port 212 and the second inflow port 221 of the cartridge for culture medium replacement use 200 are respectively connected to the inflow port and the outflow port of the culture container 75. Subsequently, the valve 216 between the liquid storage chamber 213 and the first outflow port 212, the valve 217 between the liquid storage chamber 213 and the ventilation port 214, the valve 225 between the second inflow port 221 and the curved flow path 223, and the valve 226 between the curved flow path 223 and the second outflow port 222 are respectively opened. The curved flow path 223 is depressurized by the depressurization pump via the second outflow port 222. As a result, the old culture medium in the culture container 75 flows out from the second inflow port 221 into the curved flow path 223, and the new culture medium in the liquid storage chamber 213 flows into the culture container 75 from the first outflow port 212. Thereafter, the valve 225 between the second outflow port 222 and the curved flow path 223 and the valve 226 between the curved flow path 223 and the second outflow port 222 are respectively closed, and the curved flow path 223 is hermetically sealed. The valve driving mechanism 249 is installed in the culture medium replacement part 103. The valves 216, 217, 225 and 226 operated here are opened and closed by the valve driving mechanism 249.

In the present embodiment, the culture medium replacement operation is performed while the culture container 75 is accommodated in the incubator part 101 without being taken out from the incubator part 101. Therefore, it is possible to remarkably reduce the environmental variation with respect to the cells existing in the culture container 75.

Subsequently, as shown in FIG. 4, by means of the cartridge conveying part 109, the cartridge for culture medium replacement use 200 is separated from the culture medium replacement part 103 and the culture container 75 in a state in which the culture medium is accommodated in the curved flow path 223, and is moved to the culture medium collection part 104.

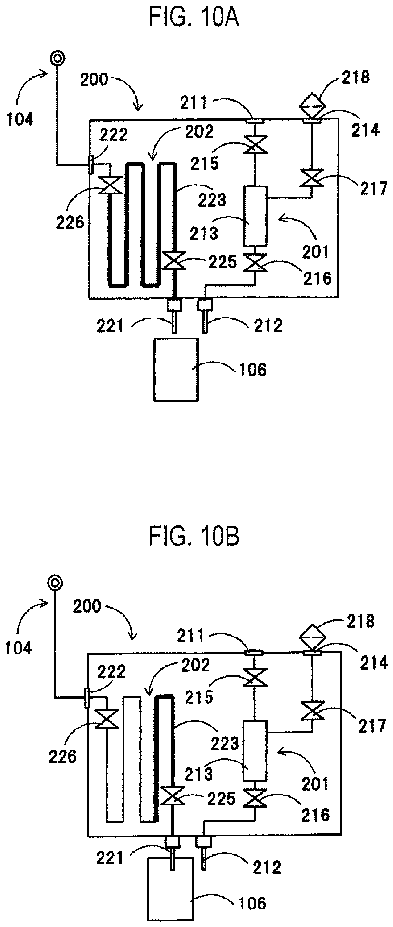

In the culture medium collection part 104, as shown in FIG. 10A, a pressurization pump (not shown) is connected to the second outflow port 222 of the cartridge for culture medium replacement use 200. In order to supply a new culture medium to the culture container 75 and completely replace the interior of the culture container 75 with the new culture medium, it is preferable that the volume of the culture medium supplied to the culture container 75 is larger than the volume of the culture container 75. In this regard, when the volume of the new culture medium to be supplied to the culture container 75 is larger than the volume of the culture container 75, the new culture medium supplied from the liquid storage chamber 213 to the culture container 75 is accommodated at the side of the second inflow port 221 of the curved flow path 223. In addition, the new culture medium and the old culture medium may be partially mixed with each other in the boundary between the new culture medium and the old culture medium. Therefore, the valve 226 between the second outflow port 222 and the curved flow path 223 and the valve 225 between the curved flow path 223 and the second inflow port 221 are respectively opened. The curved flow path 223 is pressurized from the side of the second outflow port 222 by the pressurization pump. The new culture medium accommodated at the side of the second inflow port 221 of the curved flow path 223 and the mixture of the new culture medium and the old culture medium are pushed out and discharged from the second inflow port 221 to the outside. After the entire culture medium containing the new culture medium is discharged from the second inflow port 221, the pressurization performed by the pressurization pump is stopped.

Subsequently, as shown in FIG. 10B, the culture medium analysis part 106 is connected to the second inflow port 221 of the cartridge for culture medium replacement use 200. Thereafter, in a state in which the valve 226 between the second outflow port 222 and the curved flow path 223 and the valve 225 between the curved flow path 223 and the second inflow port 221 are respectively opened, the curved flow path 223 is again pressurized again from the side of the second outflow port 222 by the pressurization pump. As a result, the old culture medium in the curved flow path 223 is pushed out from the second inflow port 221 toward the culture medium analysis part 106 and is collected. The culture medium analysis part 106 analyzes the components of the old culture medium thus collected. The valve driving mechanism 249 is installed in the culture medium collection part 104. The valves 225 and 226 operated here are opened and closed by the valve driving mechanism 249.

Subsequently, as shown in FIG. 4, by means of the cartridge conveying part 109, the cartridge for culture medium replacement use 200 is separated from the culture medium collection part 104 and the culture medium analysis part 106 and is moved to the flow path cleaning part 105.

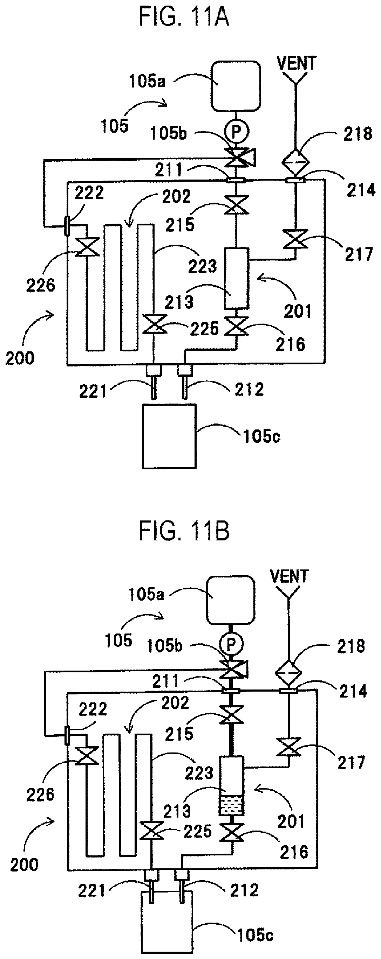

In the flow path cleaning part 105, as shown in FIG. 11A, a cleaning liquid storage container 105a is coupled to the first inflow port 211 and the second outflow port 222 of the cartridge for culture medium replacement use 200 via a three-way valve 105b. Subsequently, as shown in FIG. 11B, the valve 215 between the first inflow port 211 and the liquid storage chamber 213 and the valve 217 between the liquid storage chamber 213 and the ventilation port 214 are respectively opened. On the other hand, the valve 216 between the liquid storage chamber 213 and the first outflow port 212 is closed. In this state, the cleaning liquid storage container 105a and the first inflow port 211 are brought into communication with each other by the three-way valve 105b so that a cleaning liquid is supplied from the cleaning liquid storage container 105a via the first inflow port 211. Since the valve 216 between the liquid storage chamber 213 and the first outflow port 212 is closed, the supplied cleaning liquid is retained inside the liquid storage chamber 213. After the liquid storage chamber 213 is filled with the cleaning liquid, the flow path between the cleaning liquid storage container 105a and the first inflow port 211 is closed by the three-way valve 105b.

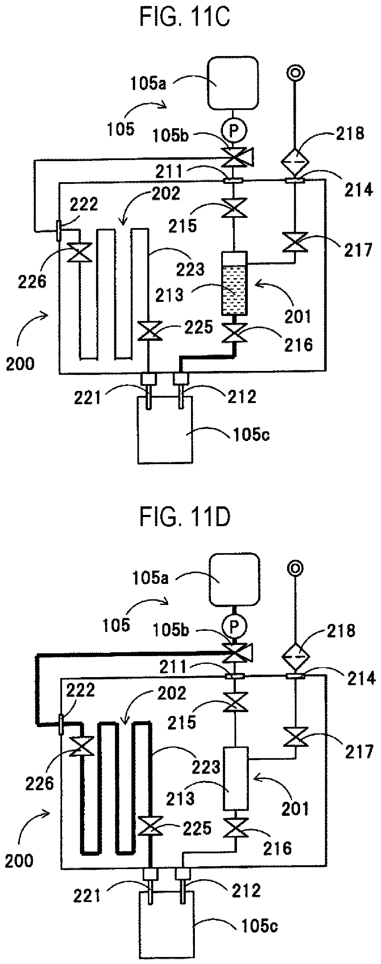

Subsequently, as shown in FIG. 11C, a waste liquid container 105c is connected to the first inflow port 211 and the second outflow port 222 of the cartridge for culture medium replacement use 200. A pressurization pump (not shown) is connected to the ventilation port 214. Thereafter, the valve 216 between the liquid storage chamber 213 and the first outflow port 212 is opened. The interior of the liquid storage chamber 213 is pressurized by the pressurization pump so that the cleaning liquid inside the liquid storage chamber 213 is pushed out and discharged from the first outflow port 212 to the waste liquid container 105c.

Subsequently, as shown in FIG. 11D, the valve 226 between the second outflow port 222 and the curved flow path 223 and the valve 225 between the curved flow path 223 and the second inflow port 221 are respectively opened. In this state, the cleaning liquid storage container 105a and the second outflow port 222 are brought into communication with each other by the three-way valve 105b so that the cleaning liquid is supplied from the cleaning liquid storage container 105a via the second outflow port 222. The cleaning liquid supplied from the second outflow port 222 passes through the curved flow path 223 and is discharged from the second inflow port 221 to the waste liquid container 105c. The valve driving mechanism 249 is installed in the flow path cleaning part 105. The valves 215, 216, 217, 225 and 226 operated here are opened and closed by the valve driving mechanism 249.

In this example, the liquid collection flow path 202 is cleaned after the liquid supply flow path 201 is cleaned. However, the liquid supply flow path 201 may be cleaned after the liquid collection flow path 202 is cleaned.

Subsequently, as shown in FIG. 4, by means of the cartridge conveying part 109, the cartridge for culture medium replacement use 200 thus cleaned is separated from the flow path cleaning part 105 and is returned to the culture medium supply part 102. In this way, the cartridge for culture medium replacement use 200 is reused for a subsequent culture medium replacement.

If the cartridge for culture medium replacement use 200 is repeatedly reused, proteins gradually adhere to and contaminate the liquid supply flow path 201 and the liquid collection flow path 202. Therefore, by means of the cartridge conveying part 109, the used cartridge for culture medium replacement use 200 is periodically discharged to the cartridge collection part 108, and the unused cartridge for culture medium replacement use 200 is periodically taken out from the cartridge storage part 107.

### <Effect>

Next, descriptions will be made on the effects which are achieved by the present embodiment having the above-described configuration and which have not yet been described, or the effects which are particularly important.

The cartridge for culture medium replacement use 200 capable of accommodating a predetermined amount of liquid culture medium moves between the culture medium supply part 102, the culture medium replacement part 103 and the culture medium collection part 104, whereby the culture medium replacement can be performed in a state in which the culture container 75 is separated from the culture medium supply part 102 and the culture medium collection part 104. This eliminates the need to centrally arrange other devices such as the medium storage container 102a and the collection container around the culture container 75. Thus, the liquid flow paths are not made redundant due to the physical limitation caused by device concentration. Accordingly, as compared with the conventional culture medium replacement mechanism, it is possible to shorten the liquid flow path.

Furthermore, the liquid flow paths are formed in the cartridge for culture medium replacement use 200, namely a cartridge-like part. Thus, the cartridge for culture medium replacement use 200 can be easily gripped and carried even by a commercially available robot hand. This makes it easy to automate the replacement of the liquid flow paths.

Moreover, by using a plurality of cartridges for culture medium replacement use 200, it is possible to simultaneously perform respective processes such as a culture medium supply, culture medium replacement, culture medium collection, and flow path cleaning in parallel. Therefore, it is possible to restrain the working time from being prolonged in the case of processing a plurality of culture containers 75.

Furthermore, by performing the culture medium replacement while the culture container 75 is accommodated in the incubator part 101, it is possible to remarkably reduce the environmental variation with respect to the cells existing in the culture container 75. It is not necessarily essential that the culture medium replacement is performed while the culture container 75 is accommodated in the incubator part 101. The culture container 75 may be taken out from the incubator part 101 and the culture medium replacement may be performed on a culture medium replacement stage (not shown).

In addition, the liquid collection flow path 202 of the cartridge for culture medium replacement use 200 includes the curved flow path 223 having a small flow path diameter and an increased flow path length. It is therefore possible to collect the old culture medium flowing out from the culture container 75 so as not to be mixed with the new culture medium along the curved flow path 223. As a result, the old culture medium can be discriminated from the new culture medium and can be discharged from the curved flow path 223. The components of the old culture medium old medium used inside the culture container 75 can be accurately analyzed.

### <Modification>

Various modifications can be made with respect to the above-described embodiment. Hereinafter, modifications will be described with reference to the drawings. In the following descriptions and the drawings used in the following descriptions, the same reference numerals as those used for the corresponding parts in the above-described embodiment are used for the parts that can be configured similarly to the above-described embodiment. Duplicate descriptions will be omitted. In addition, when it is obvious that the operations and effects obtained in the above-described embodiment can be obtained in the modification, the descriptions thereof may be omitted.

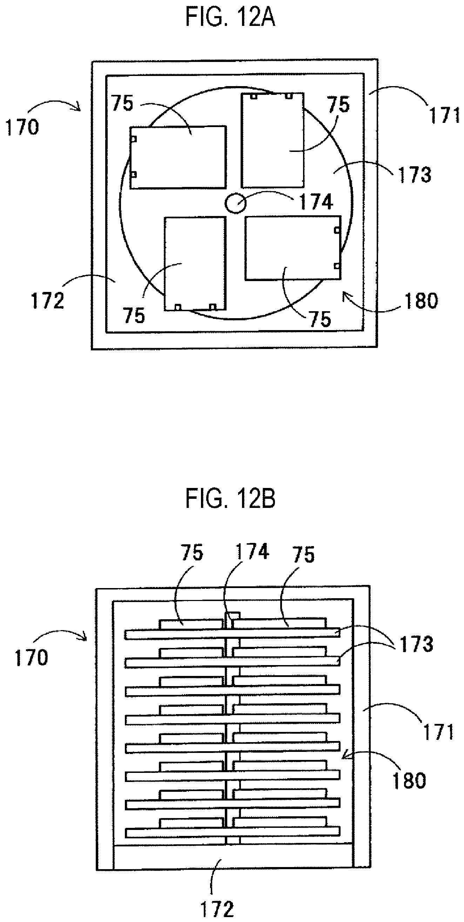

FIG. 12A is an internal top view showing a configuration of a first modification of the transport container capable of accommodating a plurality of culture containers 75, and FIG. 12B is an internal side view of the transport container shown in FIG. 12A.

The transport container 170 shown in FIG. 12A and FIG. 12B includes a housing 171 and a cassette 180 capable of holding a plurality of culture containers 75. Among these, the housing 171 has a rectangular tubular shape with its lower end portion opened. On the other hand, the cassette 180 includes a lid body 172 fitted to the opening of the lower end portion of the housing 171 to hermetically seal the housing 171, a support column 174 installed to extend vertically upward from the lid body 172, and a plurality of (eight, in the illustrated example) shelves 173 installed side by side along the support column 174.

Each of the shelves 173 has a disc shape and is fixed to the support column 174 in a horizontally oriented posture. On each of the shelves 173, a plurality of (four, in the illustrated example) culture containers 75 are mounted in a rotational symmetry relationship (quadruple symmetry relationship, in the illustrated example) around the support column 174.

Similar to the transport container 70 shown in FIG. 3, the transport container 170 shown in FIG. 12A and FIG. 12B is transported along the rail 65 to the loading part 21 of the iPS cell automatic culture devices 20, the loading part 31 of the differentiated cell automatic culture devices 30, or the loading part 12a of the iPS cell establishing device 11, in a state in which the transport container 170 is suspended downward by the holding part 61.

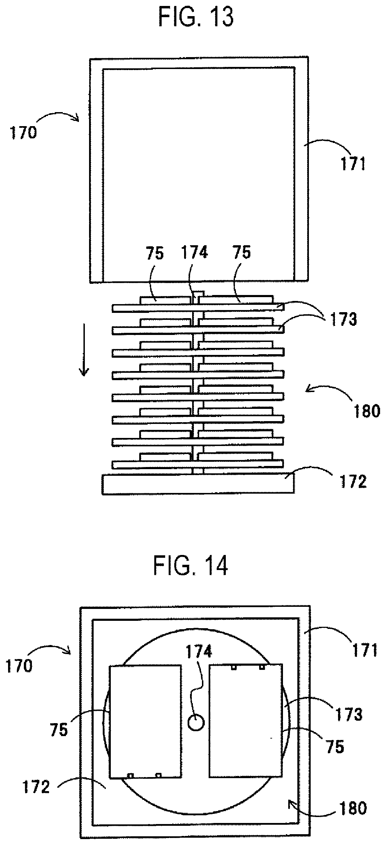

When taking out the culture container 75 from this transport container 170, as shown in FIG. 13, the cassette 180 is pulled downward by a rotary elevator mechanism (not shown) and is taken outside of the housing 171 in a state in which the plurality of culture containers 75 is held in the cassette 180. Then, one culture container 75 is gripped and taken out from the upper side of one shelf 173 by a robot hand (not shown). After the culture containers 75 are taken out one by one from the upper side of each of the shelves 173, the cassette 180 is rotated by 90 degrees around the support column 174 by the rotary elevator mechanism. Then, the subsequent culture container 75 is gripped and taken out from the upper side of one shelf 173 by the robot hand. By repeating the taking-out of the culture container 75 using the robot hand and the rotation of the cassette 180 using the rotary elevator mechanism, all the culture containers 75 held in the cassette 180 can be taken out by the robot hand.

According to the transport container 170 configured as above, the plurality of culture containers 75 can be mounted on each of the plurality of shelves 173. It is therefore possible to increase the number of culture containers 75 accommodated in the transport container 170 as compared with the transport container 70 shown in FIG. 3. This makes it possible to collectively transport a larger number of culture containers 75 into the device.

Furthermore, the plurality of culture containers 75 mounted on each of the shelves 173 is mounted in a rotational symmetry relationship around the support column 174. Therefore, by rotating the cassette 180 about the support column 174, access points of the culture containers 175 accessed by the robot hand can be made common to one point. This makes it possible to simplify the container taking-out mechanism.

In the example shown in FIGS. 12A and 12B, four culture containers 75 are mounted on each shelf 173. However, the present disclosure is not limited thereto. For example, as shown in FIG. 14, two culture containers 75 may be mounted on each shelf 173 in a double symmetry relationship about the support column 174. Alternatively, n (n=3, 5, 6, 7...) culture containers 75 may be mounted on each shelf 173 in an n symmetry relationship around the support column 174.

Next, a modification of the cell culture device used together with the transport container 170 shown in FIGS. 12A and 12B will be described.

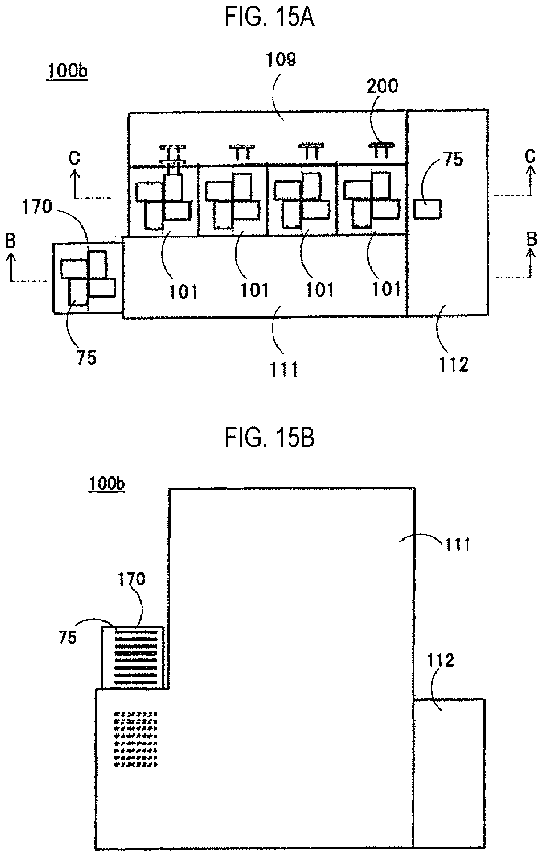

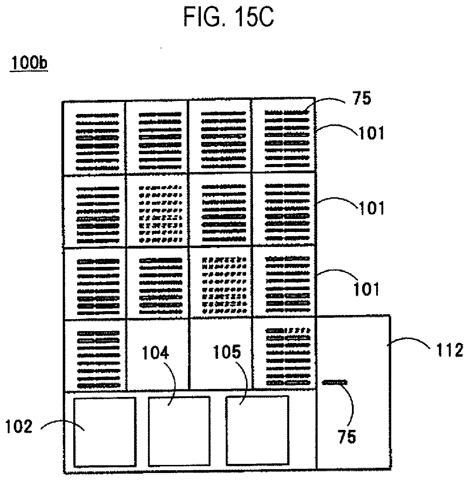

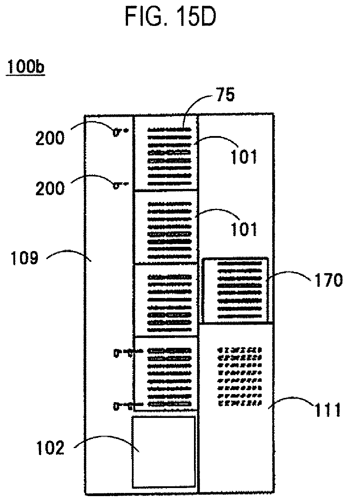

FIG. 15A is a schematic plan view showing a configuration of a modification of the cell culture device. FIG. 15B is a sectional view of the cell culture device taken along line B-B in FIG. 15A. FIG. 15C is a sectional view of the cell culture device taken along line C-C in FIG. 15A. FIG. 15D is a left side view of the cell culture device shown in FIG. 15A.

The cell culture device 100b shown in FIGS. 15A to 15D includes an in-device transfer part 111 configured to take out a cassette 180 (not shown in FIGS. 15A to 15D) holding a plurality of culture containers 75 from a transport container 170 shown in FIGS. 12A and 12B and configured to transfer the cassette 180 holding the plurality of culture containers 75 up to the incubator part 101, and a plurality of (sixteen, in the illustrated example) incubator parts 101 capable of accommodating the plurality of culture containers 75 while being held by the cassette 180. Similar to the cell culture device 100 shown in FIG. 4, the cell culture device 100b further includes a cartridge conveying part 109 configured to convey the cartridge for culture medium replacement use 200, a culture medium supply part 102, a culture medium replacement part (not shown in FIGS. 15A to 15D), a culture medium collection part 104, and a flow path cleaning part 105. In FIGS. 15A to 15D, only the portions of the first outflow port and the second inflow port of the cartridge for culture medium replacement use 200 connectable to the culture container 75 are shown.

As shown in FIG. 15C, the sixteen incubator parts 101 are arranged in a 4×4 matrix shape along vertical and horizontal directions. Each of the incubator parts 101 is accessible from both the in-device transfer part 111 and the cartridge conveying part 109. A turntable (not shown) is installed in each of the incubator parts 101 to rotate the cassette 180 holding the plurality of culture containers 75 around the support column 174.

In the example shown in FIG. 15C, a cell inspection removal part 112 is connected to the incubator part 101 located at the lower right side. The cell inspection removal part 112 takes out the culture containers 75 one by one from the incubator part 101 connected thereto. The cell inspection removal part 112 is configured to inspect the cells existing in the culture container 75 thus taken-out and remove the cells having a bad state.

Next, a method of using the cell culture device 100b shown in FIGS. 15A to 15D will be described.

First, the transport container 170 transferred by the container transfer part 60 is connected to the in-device transfer part 111. The cassette 180 holding the plurality of culture containers 75 is taken out from the transport container 170 to the in-device transfer part 111.

Subsequently, the in-device transfer part 111 loads the cassette 180 holding the plurality of culture containers 75 into one incubator part 101. Inside the incubator part 101, one or all of a temperature, a humidity and a gas concentration are automatically adjusted. From the viewpoint of running cost, it is preferable that the operations of other incubator parts 101 not in use are stopped at this time point.

Subsequently, a culture medium replacement using the cartridge for culture medium replacement use 200 is performed with respect to the culture container 75 accommodated in the incubator part 101 in the same manner as the above-described culture medium replacement method.

That is to say, after the cartridge for culture medium replacement use 200 conveyed by the cartridge conveying part 109 is connected to the culture medium supply part 102 and the liquid culture medium is supplied to the liquid supply flow path 201 of the cartridge for culture medium replacement use 200, the cartridge for culture medium replacement use 200 is separated from the culture medium supply part 102.

Subsequently, the cartridge for culture medium replacement use 200 conveyed by the cartridge conveying part 109 is connected to one culture container 75 mounted on one shelf 173 accommodated in the incubator part101. The liquid culture medium inside the liquid supply flow path 201 is supplied into the culture container 75, and the liquid culture medium inside the culture container 75 is collected into the liquid collection flow path 202. Thereafter, the cartridge for culture medium replacement use 200 is separated from the culture container 75.

Subsequently, the cartridge for culture medium replacement use 200 conveyed by the cartridge conveying part 109 is connected to the culture medium collection part 104. After the liquid culture medium is collected from the liquid collection flow path 202 to the culture medium collection part 104, the cartridge for culture medium replacement use 200 is separated from the culture medium collection part 104.

Subsequently, the cartridge for culture medium replacement use 200 conveyed by the cartridge conveying part 109 is connected to the flow path cleaning part 105. The liquid supply flow path 201 and the liquid collection flow path 202 are respectively cleaned. The cartridge for culture medium replacement use 200 thus cleaned is separated from the flow path cleaning part 105 and is returned to the culture medium supply part 102. The cleaned cartridge for culture medium replacement use 200 is used for the subsequent culture medium replacement.

After the culture medium replacement is performed with respect to one culture container 75 mounted on each shelf 173, the cassette 180 is rotated by 90 degrees around the support column 174 by the turntable installed in the incubator part 101. Then, the culture medium replacement is performed with respect to a subsequent culture container 75 mounted on each shelf 173.

As described above, the plurality of culture containers 75 are accommodated in the incubator part 101 in a state in which the culture containers 75 are held by the cassette 180 and the cassette 180 is rotated around the support column 174. Thus, in each shelf 173, the access points of the cartridge for culture medium replacement use 200 accessed by the cartridge conveying part 109 can be made common to one point. This makes it possible to simplify the cartridge conveying part 109.

The in-device transfer part 111 periodically transfers the cassette 180 holding the plurality of culture containers 75 to another incubator part 101 to which the cell inspection removal part 112 is connected. The cell inspection removal part 112 takes out the culture containers 75 one by one from the incubator part 101. The cell inspection removal part 112 inspects the cells existing in the culture containers 75 and removes the cells having a bad state. Then, the inspected culture containers are returned into the incubator part 101. Thereafter, the in-device transfer part 111 returns the cassette 180 holding the plurality of inspected culture containers 75 to the original incubator part 101.

Meanwhile, in the cell culturing step, it is necessary to handle the plurality of culture containers 75 in order to distribute the cells into the plurality of culture containers 75 along with the growth of the cells and to continuously culture the cells. In the case where the culture containers 75 are transferred one by one inside the device as in the conventional cell culture device, even if the cells have the same basis, a time difference occurs between the initial culture container 75 and the final culture container 75 as the culture proceeds. This greatly changes the culture conditions.

On the other hand, according to the cell culture device 100b shown in FIGS. 15A to 15D, the plurality of culture containers 75 is collectively conveyed in a state in which the culture containers 75 are held by the cassette 180. It is therefore possible to reduce a difference in culture time.

In a case where the subculture of cells are performed in the cell culture device 100b shown in FIGS. 15A to 15D, the number of incubator parts 101 to be used may be increased in conformity with the increasing number of culture vessels. By gradually increasing the number of incubator parts 101 to be used, it is possible to reduce the running cost.

### EXPLANATION OF REFERENCE NUMERALS

- 75:: culture container,
- 100, 100b:: cell culture device
- 101:: incubator part
- 102:: culture medium supply part
- 103:: culture medium replacement part
- 104:: culture medium collection part
- 105:: flow path cleaning part
- 106:: culture medium analysis part
- 107:: cartridge storage part
- 108:: cartridge collection part
- 109:: cartridge conveying part
- 200:: cartridge for culture medium replacement use
- 201:: liquid supply flow path
- 202:: liquid collection flow path
- 211:: first inflow port
- 212:: first outflow port
- 213:: liquid storage chamber
- 214:: ventilation port
- 221:: second inflow port
- 222:: second outflow port
- 223:: curved flow path
- 224:: tank-shaped flow path

## Claims

1. A cell culture device (20, 30, 100, 100b), comprising:
an incubator part (17, 27, 37, 101) configured to accommodate a closed-system culture container (75);
a cartridge for culture medium replacement use (200) having a liquid supply flow path (201) and a liquid collection flow path (202);
a culture medium supply part (102) configured to supply a liquid culture medium to the liquid supply flow path (201) of the cartridge for culture medium replacement use (200);
a culture medium replacement part (103) configured to cause the liquid culture medium existing in the liquid supply flow path (201) to flow into the culture container (75) and to cause the liquid culture medium existing in the culture container (75) to flow out to the liquid collection flow path (202) in a state in which the cartridge for culture medium replacement use (200) is connected to the culture container (75); and
a culture medium collection part (104) configured to collect the liquid culture medium from the liquid collection flow path (202) of the cartridge for culture medium replacement use (200),
wherein the cartridge for culture medium replacement use (200) is removably attachable to the closed-system culture container (75) and is movable between the culture medium supply part (102), the culture medium replacement part (103) and the culture medium collection part (104).

2. The device (20, 30, 100, 100b) of claim 1, further comprising:
a cartridge conveying part (109) configured to move the cartridge for culture medium replacement use (200) between the culture medium supply part (102), the culture medium replacement part (103) and the culture medium collection part (104).

3. The device (20, 30, 100, 100b) of claim 1 or 2, further comprising:
a flow path cleaning part (105) configured to clean the liquid supply flow path (201) and the liquid collection flow path (202) of the cartridge for culture medium replacement use (200),
wherein the cartridge for culture medium replacement use (200) is movable between the culture medium supply part (102), the culture medium replacement part (103), the culture medium collection part (104) and the flow path cleaning part (105).

4. The device (20, 30, 100, 100b) of any one of claims 1 to 3, wherein the culture medium replacement part (103) is installed adjacent to the incubator part (101), and a culture medium replacement in the culture container (75) can be performed via the cartridge for culture medium replacement use (200) while keeping the culture container (75) accommodated in the incubator part (101).

5. The device (20, 30, 100, 100b) of any one of claims 1 to 4, wherein a culture medium analysis part (34) configured to analyze a collected liquid culture medium is installed in the culture medium collection part (104).

6. The device (20, 30, 100, 100b) of any one of claims 1 to 5, further comprising:
a cartridge storage part (107) configured to store a plurality of unused cartridges for culture medium replacement use (200); and
a cartridge collection part (108) configured to collect a used cartridge for culture medium replacement use (200).

7. The device (20, 30, 100, 100b) of any one of claims 1 to 6, wherein the liquid supply flow path (201) includes a first inflow port (211) connectable to the culture medium supply part (102), a first outflow port (212) connectable to an inflow port (211) of the culture container (75), a liquid storage chamber (213) configured to bring the first inflow port (211) and the first outflow port (212) into communication with each other and a ventilation port (214) communicating with the liquid storage chamber (213), and
the liquid collection flow path (202) includes a second inflow port (221) connectable to an outflow port of the culture container (75), a second outflow port (222) connectable to the culture medium collection part (104) and a curved flow path (223) configured to bring the second inflow port (221) and the second outflow port (222) into communication with each other, the curved flow path (223) including a plurality of bent portions or curved portions.

8. The device (20, 30, 100, 100b) of claim 7, wherein the curved flow path (223) has a serpentine shape or a spiral shape.

9. The device (20, 30, 100, 100b) of claim 7 or 8, wherein the first outflow port (212) and the second inflow port (221) are formed adjacent to each other.

10. A method for replacing a culture medium, comprising:
a culture medium supply step of connecting a cartridge for culture medium replacement use (200) having a liquid supply flow path (201) and a liquid collection flow path (202) to a culture medium supply part (102), supplying a liquid culture medium from the culture medium supply part (102) to the liquid supply flow path (201), and separating the cartridge for culture medium replacement use (200) from the culture medium supply part (102);
a culture medium replacement step of connecting the cartridge for culture medium replacement use (200) to a closed-system culture container (75), causing the liquid culture medium existing in the liquid supply flow path (201) to flow into the culture container (75), causing the liquid culture medium existing in the culture container (75) to flow out to the liquid collection flow path (202), and separating the cartridge for culture medium replacement use (200) from the culture container (75); and
a culture medium collection step of connecting the cartridge for culture medium replacement use (200) to a culture medium collection part (104), collecting the liquid culture medium from the liquid collection flow path (202) to the culture medium collection part (104), and separating the cartridge for culture medium replacement use (200) from the culture medium collection part (104).

11. The method of claim 10, further comprising:
a cleaning step of cleaning the liquid supply flow path (201) and the liquid collection flow path (202) of the cartridge for culture medium replacement use (200) after the culture medium collection step.

12. The method of claim 10 or 11, wherein the culture medium replacement step is performed in a state in which the culture container (75) is accommodated in an incubator part (101).

13. The method of any one of claims 10 to 12, further comprising:
a culture medium analysis step of analyzing a collected liquid culture medium after the culture medium collection step.

## Patentansprüche

1. Zellkulturvorrichtung (20, 30, 100, 100b), mit:
einem Inkubatorteil (17, 27, 37, 101), ausgebildet zum Beherbergen eines Kulturbehälters (75) mit geschlossenem System;
einer Kartusche für die Verwendung zum Kulturmedium-Austausch (200) mit einem Flüssigkeits-Zufuhrpfad (201) und einem Flüssigkeits-Sammelpfad (202); einem Kulturmedium-Zufuhrteil (102), ausgebildet zum Zuführen eines flüssigen Kulturmediums zu dem Flüssigkeits-Zufuhrpfad (201) der Kartusche zur Verwendung zum Kulturmedium-Austausch (200);
einem Kulturmedium-Austauschteil (103) ausgebildet zum Bewirken, dass in dem Flüssigkeits-Zufuhrpfad (201) befindliches flüssiges Kulturmedium in den Kulturbehälter (75) fließt, und zum Bewirken, dass in dem Kulturbehälter (75) befindliches flüssiges Kulturmedium zu dem Flüssigkeits-Sammelpfad (202) ausfließt, wenn die Kartusche für die Verwendung zum Kulturmedium-Austausch (200) mit dem Kulturbehälter (75) verbunden wird; und
einem Kulturmedium-Aufnahmeteil (104), ausgebildet zum Aufnehmen des flüssigen Kulturmediums aus dem Flüssigkeits-Sammelpfad (202) der Kartusche für die Verwendung zum Kulturmedium-Austausch (200),
wobei die Kartusche für die Verwendung zum Kulturmedium-Austausch (200) entfernbar an dem Kulturbehälter (75) mit geschlossenem System anbringbar und zwischen dem Kulturmedium-Zufuhrteil (102), dem Kulturmedium-Austauschteil (103) und dem Kulturmedium-Aufnahmeteil (104) bewegbar ist.

2. Vorrichtung (20, 30, 100, 100b) nach Anspruch 1, ferner aufweisend:
einen Kartuschen-Bewegungsteil (109), ausgebildet zum Bewegen der Kartusche für die Verwendung zum Kulturmedium-Austausch (200) zwischen dem Kulturmedium-Zufuhrteil (102), dem Kulturmedium-Austauschteil (103) und dem Kulturmedium-Aufnahmeteil (104).

3. Vorrichtung (20, 30, 100, 100b) nach Anspruch 1 oder 2, ferner aufweisend:
einen Strömungspfad-Reinigungsteil (105), ausgebildet zum Reinigen des Flüssigkeits-Zufuhrpfads (201) und des Flüssigkeits-Sammelpfads (202) der Kartusche für die Verwendung zum Kulturmedium-Austausch (200),
wobei die Kartusche für die Verwendung zum Kulturmedium-Austausch (200) zwischen dem Kulturmedium-Zufuhrteil (102), dem Kulturmedium-Austauschteil (103), dem Kulturmedium-Aufnahmeteil (104) und dem Strömungspfad-Reinigungsteil (105) bewegbar ist.

4. Vorrichtung (20, 30, 100, 100b) nach einem der Ansprüche 1 bis 3, wobei der Kulturmedium-Austauschteil (103) benachbart zum Inkubatorteil (101) eingebaut ist, und ein Kulturmedium-Austausch in dem Kulturbehälter (75) mittels der Kartusche für die Verendung zum Kulturmedium-Austausch (200) durchgeführt werden kann, während der Kulturbehälter (75) in dem Inkubatorteil (101) beherbergt bleibt.

5. Vorrichtung (20, 30, 100, 100b) nach einem der Ansprüche 1 bis 4, wobei ein Kulturmedium-Analyseteil (34), ausgebildet zum Analysieren eines gesammelten flüssigen Kulturmediums, in dem Kulturmedium-Sammelteil (104) eingebaut ist.

6. Vorrichtung (20, 30, 100, 100b) nach einem der Ansprüche 1 bis 5, ferner aufweisend:
einen Kartuschen-Aufbewahrungsteil (107), ausgebildet zum Aufbewahren mehrerer nicht verwendeter Kartuschen für die Verwendung zum Kulturmedium-Austausch (200); und
einem Kartuschen-Sammelteil (108), ausgebildet zum Sammeln einer verwendeten Kartusche für die Verwendung zum Kulturmedium-Austausch (200).

7. Vorrichtung (20, 30, 100, 100b) nach einem der Ansprüche 1 bis 6, wobei der Flüssigkeits-Zufuhrpfad (201) eine erste Zuflussöffnung (211) aufweist, die mit dem Kulturmedium-Zufuhrteil (102) verbindbar ist, eine erste Ausflussöffnung (212), die mit einer Zuflussöffnung (211) des Kulturbehälters (75) verbindbar ist, eine Flüssigkeits-Sammelkammer (213), ausgebildet zum In-Verbindung-Bringen der ersten Zuflussöffnung (211) und der ersten Ausflussöffnung (212) miteinander, und eine Ventilationsöffnung (214), die mit der Flüssigkeits-Sammelkammer (213) kommuniziert, und
wobei der Flüssigkeits-Sammelpfad (202) eine zweite Zuflussöffnung (221) aufweist, die mit einer Ausflussöffnung des Kulturbehälters (75) verbindbar ist, eine zweite Ausflussöffnung (222), die mit dem Kulturmedium-Sammelteil (104) verbindbar ist, und einen gekrümmten Strömungspfad (223), ausgebildet zum In-Verbindung-Bringen der zweiten Zuflussöffnung (221) und der zweiten Ausflussöffnung (222) miteinander, wobei der gekrümmte Strömungspfad (223) mehrere gebogene oder gekrümmte Abschnitte aufweist.

8. Vorrichtung (20, 30, 100, 100b) nach Anspruch 7, wobei der gekrümmte Strömungspfad (223) eine geschlängelte Gestalt oder eine spiralige Gestalt aufweist.

9. Vorrichtung (20, 30, 100, 100b) nach Anspruch 7 oder 8, wobei die erste Ausflussöffnung (212) und die zweite Zuflussöffnung (221) zueinander benachbart angeordnet sind.

10. Verfahren zum Austauschen eines Kulturmediums, beinhaltend:
einen Kulturmedium-Zufuhrschritt des Verbindens einer Kartusche für die Verwendung zum Kulturmedium-Austausch (200) mit einem Flüssigkeits-Zufuhrpfad (201) und einem Flüssigkeits-Sammelpfad (202) mit einem Kulturmedium-Zufuhrteil (102), Zuführen eines flüssigen Kulturmediums aus dem Kulturmedium-Zufuhrteil (102) zu dem Flüssigkeits-Zufuhrpfad (201), und Abtrennen der Kartusche für die Verwendung zum Kulturmedium-Austausch (200) von dem Kulturmedium-Zufuhrteil (102);
einen Kulturmedium-Austauschschritt des Verbindens der Kartusche für die Verwendung zum Kulturmedium-Austausch (200) mit einem Kulturbehälter (75) mit geschlossenem System, zum Bewirken, dass das in dem Flüssigkeits-Zufuhrpfad (201) befindliche flüssige Kulturmedium in den Kulturbehälter (75) fließt, zum Bewirken, dass in dem Kulturbehälter (75) befindliches flüssiges Kulturmedium zu dem Flüssigkeits-Sammelpfad (202) ausfließt, und des Abtrennens der Kartusche für die Verwendung zum Kulturmedium-Austausch (200) von dem Kulturbehälter (75); und
einen Kulturmedium-Aufnahmeschritt des Verbindens der Kartusche für die Verwendung zum Kulturmedium-Austausch (200) mit einem Kulturmedium-Aufnahmeteil (104), Aufnehmen des flüssigen Kulturmediums aus dem Flüssigkeits-Sammelpfad (202) zu dem Kulturmedium-Sammelpfad (104), und Abtrennen der Kartusche für die Verwendung zum Kulturmedium-Austausch (200) von dem Kulturmedium-Aufnahmeteil (104).

11. Verfahren nach Anspruch 10, ferner beinhaltend:
einen Reinigungsschritt des Reinigens des Flüssigkeits-Zufuhrpfads (201) und des Flüssigkeits-Sammelpfads (202) der Kartusche für die Verwendung zum Kulturmedium-Austausch (200) nach dem Kulturmedium-Aufnahmeschritt.

12. Verfahren nach Anspruch 10 oder 11, wobei der Kulturmedium-Austauschschritt dann ausgeführt wird, wenn der Kulturbehälter (75) in einem Inkubatorteil (101) beherbergt ist.

13. Verfahren nach einem der Ansprüche 10 bis 12, ferner aufweisend:
einen Kulturmedium-Analyseschritt des Analysierens eines aufgenommenen flüssigen Kulturmediums nach dem Kulturmedium-Aufnahmeschritt.

## Revendications

1. Dispositif de culture cellulaire (20, 30, 100, 100b) comprenant :
une partie formant incubateur (17, 27, 37, 101) configurée pour loger un récipient de culture à système fermé (75) ;
une cartouche pour utilisation de remplacement de milieu de culture (200) ayant une voie d'écoulement d'alimentation en liquide (201) et une voie d'écoulement de collecte de liquide (202) ;
une partie d'alimentation en milieu de culture (102) configurée pour amener un milieu de culture liquide dans la voie d'écoulement d'alimentation en liquide (201) de la cartouche pour utilisation de remplacement de milieu de culture (200) ;
une partie de remplacement de milieu de culture (103) configurée pour amener le milieu de culture liquide existant dans la voie d'écoulement d'alimentation en liquide (201) à entrer dans le récipient de culture (75) et pour amener le milieu de culture liquide existant dans le récipient de culture (75) à sortir dans la voie d'écoulement de collecte de liquide (202) dans un état où la cartouche pour utilisation de remplacement de milieu de culture (200) est reliée au récipient de culture (75) ; et
une partie de collecte de milieu de culture (104) configurée pour collecter le milieu de culture liquide à partir de la voie d'écoulement de collecte de liquide (202) de la cartouche pour utilisation de remplacement de milieu de culture (200),
dans lequel la cartouche pour utilisation de remplacement de milieu de culture (200) est apte à être fixée de manière amovible au récipient de culture à système fermé (75) et est mobile entre la partie d'alimentation en milieu de culture (102), la partie de remplacement de milieu de culture (103) et la partie de collecte de milieu de culture (104).

2. Dispositif (20, 30, 100, 100b) de la revendication 1, comprenant également :
une partie de transport de cartouche (109) configurée pour déplacer la cartouche pour utilisation de remplacement de milieu de culture (200) entre la partie d'alimentation en milieu de culture (102), la partie de remplacement de milieu de culture (103) et la partie de collecte de milieu de culture (104).

3. Dispositif (20, 30, 100, 100b) de la revendication 1 ou 2, comprenant également :
une partie de nettoyage de voie d'écoulement (105) configurée pour nettoyer la voie d'écoulement d'alimentation en liquide (201) et la voie d'écoulement de collecte de liquide (202) de la cartouche pour utilisation de remplacement de milieu de culture (200),
dans lequel la cartouche pour utilisation de remplacement de milieu de culture (200) est mobile entre la partie d'alimentation en milieu de culture (102), la partie de remplacement de milieu de culture (103), la partie de collecte de milieu de culture (104) et la partie de nettoyage de voie d'écoulement (105).

4. Dispositif (20, 30, 100, 100b) de l'une quelconque des revendications 1 à 3, dans lequel la partie de remplacement de milieu de culture (103) est installée près de la partie formant incubateur (101), et un remplacement de milieu de culture dans le récipient de culture (75) peut être réalisé via la cartouche pour utilisation de remplacement de milieu de culture (200) tout en maintenant le récipient de culture (75) logé dans la partie formant incubateur (101).

5. Dispositif (20, 30, 100, 100b) de l'une quelconque des revendications 1 à 4, dans lequel une partie d'analyse de milieu de culture (34) configurée pour analyser un milieu de culture de liquide collecté est installée dans la partie de collecte de milieu de culture (104).

6. Dispositif (20, 30, 100, 100b) de l'une quelconque des revendications 1 à 5, comprenant également :
une partie de stockage de cartouches (107) configurée pour stocker une pluralité de cartouches pour utilisation de remplacement de milieu de culture (200) non utilisées ; et
une partie de collecte de cartouche (108) configurée pour collecter une cartouche pour utilisation de remplacement de milieu de culture (200) usagée.

7. Dispositif (20, 30, 100, 100b) de l'une quelconque des revendications 1 à 6, dans lequel la voie d'écoulement d'alimentation en liquide (201) comprend un premier orifice d'entrée (211) apte à être relié à la partie d'alimentation en milieu de culture (102), un premier orifice de sortie (212) apte à être relié à un orifice d'entrée (211) du récipient de culture (75), une chambre de stockage de liquide (213) configurée pour mettre le premier orifice d'entrée (211) et le premier orifice de sortie (212) en communication l'un avec l'autre, et un orifice de ventilation (214) communiquant avec la chambre de stockage de liquide (213), et
la voie d'écoulement de collecte de liquide (202) comprend un deuxième orifice d'entrée (221) apte à être relié à un orifice de sortie du récipient de culture (75), un deuxième orifice de sortie (222) apte à être relié à la partie de collecte de milieu de culture (104), et une voie d'écoulement courbe (223) configurée pour mettre le deuxième orifice d'entrée (221) et le deuxième orifice de sortie (222) en communication l'un avec l'autre, la voie d'écoulement courbe (223) comprenant une pluralité de parties coudées ou courbes.

8. Dispositif (20, 30, 100, 100b) de la revendication 7, dans lequel la voie d'écoulement courbe (223) a une forme sinueuse ou une forme en spirale.

9. Dispositif (20, 30, 100, 100b) de la revendication 7 ou 8, dans lequel le premier orifice de sortie (212) et le deuxième orifice d'entrée (221) sont formés adjacents l'un à l'autre.

10. Méthode pour remplacer un milieu de culture, comprenant :
une étape d'alimentation en milieu de culture qui relie une cartouche pour utilisation de remplacement de milieu de culture (200) comportant une voie d'écoulement d'alimentation en liquide (201) et une voie d'écoulement de collecte de liquide (202) à une partie d'alimentation en milieu de culture (102), qui amène un milieu de culture liquide de la partie d'alimentation en milieu de culture (102) jusqu'à la voie d'écoulement d'alimentation en liquide (201), et qui sépare la cartouche pour utilisation de remplacement de milieu de culture (200) de la partie d'alimentation en milieu de culture (102) ;
une étape de remplacement de milieu de culture qui relie la cartouche pour utilisation de remplacement de milieu de culture (200) à un récipient de culture à système fermé (75), qui amène le milieu de culture liquide existant dans la voie d'écoulement d'alimentation en liquide (201) à entrer dans le récipient de culture (75), qui amène le milieu de culture liquide existant dans le récipient de culture (75) à sortir dans la voie d'écoulement de collecte de liquide (202), et qui sépare la cartouche pour utilisation de remplacement de milieu de culture (200) du récipient de culture (75) ; et
une étape de collecte de milieu de culture qui relie la cartouche pour utilisation de remplacement de milieu de culture (200) à une partie de collecte de milieu de culture (104), qui collecte le milieu de culture liquide à partir de la voie d'écoulement de collecte de liquide (202) jusqu'à la partie de collecte de milieu de culture (104), et qui sépare la cartouche pour utilisation de remplacement de milieu de culture (200) de la partie de collecte de milieu de culture (104).

11. Méthode de la revendication 10, comprenant également :
une étape de nettoyage de voie d'écoulement qui nettoie la voie d'écoulement d'alimentation en liquide (201) et la voie d'écoulement de collecte de liquide (202) de la cartouche pour utilisation de remplacement de milieu de culture (200) après l'étape de collecte de milieu de culture.

12. Méthode de la revendication 10 ou 11, dans laquelle l'étape de remplacement de milieu de culture est réalisée dans un état où le récipient de culture (75) est logé dans une partie formant incubateur (101).

13. Méthode de l'une quelconque des revendications 10 à 12, comprenant également :
une étape d'analyse de milieu de culture qui analyse un milieu de culture de liquide collecté, après l'étape de collecte de milieu de culture.
